# EUROPEAN PATENT SPECIFICATION

(11) **EP 4 059 494 B1**
(45) Date of publication and mention of the grant of the patent: **15.01.2025**
(21) Application number: 22151857.4
(22) Date of filing: 17.01.2022
(51) Int. Cl.: A61K 31/164, A61K 9/20, A61P 35/00, A61K 9/00, A61K 9/02, A61K 9/06, A61K 9/16, A61K 9/48, A61K 47/14, A61K 47/44

(54) **N-PALMITOYL-ETHANOLAMIDE FOR USE IN THE PREVENTION OF COLORECTAL CARCINOMA**
N-PALMITOYL-ETHANOLAMID ZUR VERWENDUNG BEI DER VORBEUGUNG KOLOREKTALER KARZINOME
N-PALMITOYL-ÉTHANOLAMIDE À UTILISER DANS LA PRÉVENTION DU CARCINOME COLORECTAL

(30) Priority: 18.03.2021 IT 202100006494
(43) Date of publication of application: 21.09.2022
(73) Proprietor: EPITECH GROUP S.p.A., 20144 Milano (MI) (IT)
(72) Inventor: DELLA VALLE, Francesco, deceased (IT); DELLA VALLE, Maria Federica, I-20144 MILANO (IT); GOMIERO, Chiara, I-20144 MILANO (IT); BORRELLI, Francesca, I-20144 MILANO (IT); CAPASSO, Raffaele, I-20144 MILANO (IT); IZZO, Angelo Antonio, I-20144 (IT); PAGANO, Ester, I-20144 Milano (IT); ROMANO, Barbara, I-20144 (IT)
(74) Representative: Long, Giorgio

(56) References cited:
- WO-A1-2011/027373
- GIOVANNI SARNELLI ET AL: "Palmitoylethanolamide Modulates Inflammation-Associated Vascular Endothelial Growth Factor (VEGF) Signaling via the Akt/mTOR Pathway in a Selective Peroxisome Proliferator-Activated Receptor Alpha (PPAR- )-Dependent Manner", PLOS ONE, vol. 11, no. 5, 24 May 2016 (2016-05-24), pages e0156198, XP055523221, DOI: 10.1371/journal.pone.0156198
- DATABASE Embase [online] 1 August 2021 (2021-08-01), PAGANO E. ET AL: "Key role of palmitoylethanolamide in experimental colon carcinogenesis", XP055860166, retrieved from https://onlinelibrary.wiley.com/doi/full/10.1111/bcpt.13084 Database accession no. EMB-623864476
- SARNELLI GIOVANNI ET AL: "Palmitoylethanolamide Exerts Antiproliferative Effect and Downregulates VEGF Signaling in Caco-2 Human Colon Carcinoma Cell Line Through a Selective PPAR-[alpha]-Dependent Inhibition of Akt/mTOR Pathway : PEA Inhibits Akt/mTOR Pathway in Human Adenocarcinoma Cell Caco-2", PHYSIOTHERAPY RESEARCH, vol. 30, no. 6, 1 March 2016 (2016-03-01), GB, pages 963 - 970, XP055860163, ISSN: 0951-418X, DOI: 10.1002/ptr.5601
- CORDARO MARIKA ET AL: "An Update of Palmitoylethanolamide and Luteolin Effects in Preclinical and Clinical Studies of Neuroinflammatory Events", ANTIOXIDANTS, vol. 9, no. 3, 5 March 2020 (2020-03-05), pages 216, XP055860015, DOI: 10.3390/antiox9030216
- MORINISHI TATSUYA ET AL: "Activation and Expression of Peroxisome Proliferator-Activated Receptor Alpha Are Associated with Tumorigenesis in Colorectal Carcinoma", vol. 2019, 3 July 2019 (2019-07-03), US, pages 1 - 9, XP055860153, ISSN: 1687-4757, Retrieved from the Internet <URL:https://www.ncbi.nlm.nih.gov/pmc/articles/PMC6636540/pdf/PPAR2019-7486727.pdf> DOI: 10.1155/2019/7486727
- COUCH DANIEL G. ET AL: "Cannabidiol and palmitoylethanolamide are anti-inflammatory in the acutely inflamed human colon", vol. 131, no. 21, 1 November 2017 (2017-11-01), GB, pages 2611 - 2626, XP055860695, ISSN: 0143-5221, Retrieved from the Internet <URL:https://portlandpress.com/clinsci/article/131/21/2611/71677/Cannabidiol-and-palmitoylethanolamide-are-anti> DOI: 10.1042/CS20171288
- PAGANO ESTER ET AL: "Palmitoylethanolamide Reduces Colon Cancer Cell Proliferation and Migration, Influences Tumor Cell Cycle and Exerts In Vivo Chemopreventive Effects", vol. 13, no. 8, 16 April 2021 (2021-04-16), pages 1 - 14, XP055860010, Retrieved from the Internet <URL:https://www.ncbi.nlm.nih.gov/pmc/articles/PMC8073478/pdf/cancers-13-01923.pdf> DOI: 10.3390/cancers13081923
- PILEGGI CLAUDIA ET AL: "Information about management of chronic drug therapies prescribed at hospital discharge: does it affect patients' knowledge and self-confidence?", BMC HEALTH SERVICES RESEARCH, vol. 18, no. 1, 1 December 2018 (2018-12-01), XP093072456, Retrieved from the Internet <URL:https://www.ncbi.nlm.nih.gov/pmc/articles/PMC5809870/pdf/12913_2018_Article_2895.pdf> DOI: 10.1186/s12913-018-2895-2
- AL SHOYAIB ABDULLAH ET AL: "Intraperitoneal Route of Drug Administration: Should it Be Used in Experimental Animal Studies?", ENVIRONMENTAL SCIENCE AND POLLUTION RESEARCH, SPRINGER BERLIN HEIDELBERG, BERLIN/HEIDELBERG, vol. 37, no. 1, 23 December 2019 (2019-12-23), XP036969124, ISSN: 0724-8741, [retrieved on 20191223], DOI: 10.1007/S11095-019-2745-X

## Description

### Technical field of the invention

The present disclosure relates to the use of N-palmitoylethanolamide in the context of an antitumor therapy. In particular, the invention relates to palmitoylethanolamide (PEA) for use in the prevention of colorectal carcinoma, adenomatous polyps and/or pre-neoplastic ACF (Aberrant Crypts Foci) lesions, as defined in the claims.

### Background art

Colorectal cancer (CRC) is a very common neoplastic disease and is a leading cause of death with a rapidly increasing incidence rate worldwide. It was estimated that 147,950 individuals received a new diagnosis of CRC in the United States in 2020, with 17,930 new cases (12%) in individuals under the age of 50. In Europe, CRC was second in the ranking of cancer deaths in 2018.

It has been estimated that every year, about 53,000 new diagnoses of colorectal cancer (CRC) are formulated in Italy and this disease is in second place, in order of frequency, in both men and women. Since the mid-2000s, the incidence of CRC has steadily increased due to the increase in risk factors favoring the development thereof.

Many factors trigger the onset mechanisms of this tumor, including lifestyle, unbalanced diet, genetic factors, alterations in the intestinal microbiota, inflammatory bowel diseases (IBD) and other diseases such as Crohn's disease, ulcerative colitis, familial adenomatous polyposis (FAP) and Lynch syndrome.

The incidence of CRC began to decrease coinciding with the introduction of screening and drug-prevention or chemo-prevention programs consisting of the use of substances which have been shown to reduce the risk of solid tumors appearing.

Chemo-prevention is that branch of oncology which is aimed at healthy people who may have an increased risk of developing cancer and tries to act in that broad time window during which the molecular alteration processes which cause cancer to occur. For this reason, preventive strategies aim to eliminate or reduce exposure to carcinogens, to identify subjects at risk and to intervene to interrupt the process of carcinogenesis, with a pharmacological and/or dietary action. Chemo-prevention tries to hinder neoplastic transformation by acting on both the activation phase and on proliferation.

The difference with respect to chemotherapy is very great, whereby the two approaches must not be confused: preventive treatment involves the use of substances which do not have harmful effects on healthy cells and therefore on the health of the person subjected to them. Conversely, in chemotherapy treatment, harmful chemicals are used which are directed not only at diseased cells, but also healthy ones. Therefore, chemo-prevention has the purpose of creating and maintaining a constant homeostatic balance to prevent the uncontrolled reproduction of cells.

CRC in humans occurs in the final stretch of the digestive tract and is due to the malignant transformation of polyps following the uncontrolled proliferation of intestinal mucosal cells. Polyps are considered precancerous forms, although they fall under benign pathologies.

In most cases, CRC begins as a benign adenomatous polyp from which a highly dysplastic adenoma develops which, in turn, progresses to invasive cancer due to the accumulation of genetic and epigenetic alterations which determine the progressive loss of genetic stability.

The transformation process of intestinal mucosal cells from normal to cancer in the colorectal area occurs due to mutations in the tumor suppressor genes used to block the progression of the cell cycle, protecting the cell from the accumulation of potentially cancerous mutations. When such a block is missing, the cell progresses towards transformation into a cancer cell in an uncontrolled manner.

To date, the therapy aimed at eradicating CRC is surgery, often preceded by chemo and radiotherapy in order to carry out increasingly conservative interventions and, when possible, to avoid the creation of the so-called ostomy. The most active cancer drugs in the treatment of CRC comprise fluoropyrimidines (intravenous 5-fluorouracil, oral capecitabine), oxaliplatin and irinotecan. Sarnelli et al. in Plos One, 11(5), 2016, e0156198, disclose that palmitoylethanolamide (PEA) may improve inflammation-driven angiogenesis in colonic mucosa, thus reducing the mucosal damage and potentially affecting disease progression and the shift towards carcinogenesis.

For what has been said above, particularly in relation to the genesis of colorectal carcinoma, it is therefore evident that there is a need to provide a therapy which is capable of preventing the onset of such a tumor form and which, in the case of subjects already suffering from such disease, provide a safe treatment, i.e., one which selectively acts only on cancer cells and does not affect healthy cells.

### Summary of the invention

The present invention derives from the surprising discovery that palmitoylethanolamide (PEA), preferably if used in ultra-micronized form, is capable of exerting a preventive effect on the formation of colorectal carcinoma (CRC), as well as on the formation of benign adenomatous polyps, and a specific antineoplastic effect on tumor cells.

The present invention relates to Palmitoylethanolamide for use in the prevention of colorectal carcinoma, adenomatous polyps and/or pre-neoplastic ACF lesions, wherein said palmitoylethanolamide is acting on both the activation and proliferation phase and wherein palmitoylethanolamide is contained in a pharmaceutical or veterinary formulation and is formulated in dosage forms for oral administration.

In a non-claimed aspect, the disclosure further relates to Palmitoylethanolamide for use in the antineoplastic therapy of colorectal carcinoma, in which said palmitoylethanolamide exerts a selective antiproliferative action on tumor cells and not on healthy cells.

These and further objects, as outlined in the appended claims, will be described in the following description. The text of the claims must be considered included in the description for the purpose of assessing the sufficiency of the description.

Further features and advantages of the invention will become apparent from the following description of preferred embodiments, given by way of non-limiting example.

### Brief description of the drawings

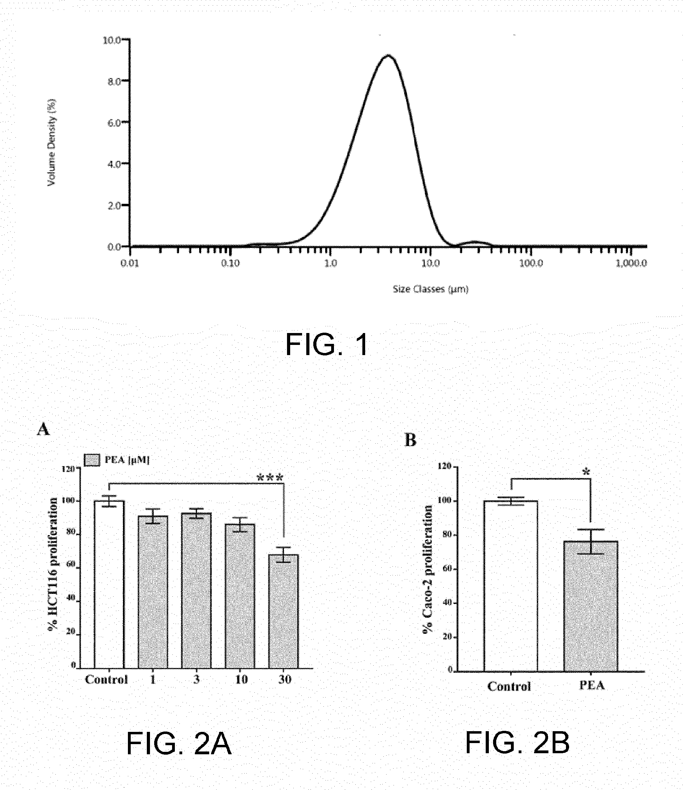

Figure 1 shows a particle size distribution graph of palmitoylethanolamide in ultra-micronized form;

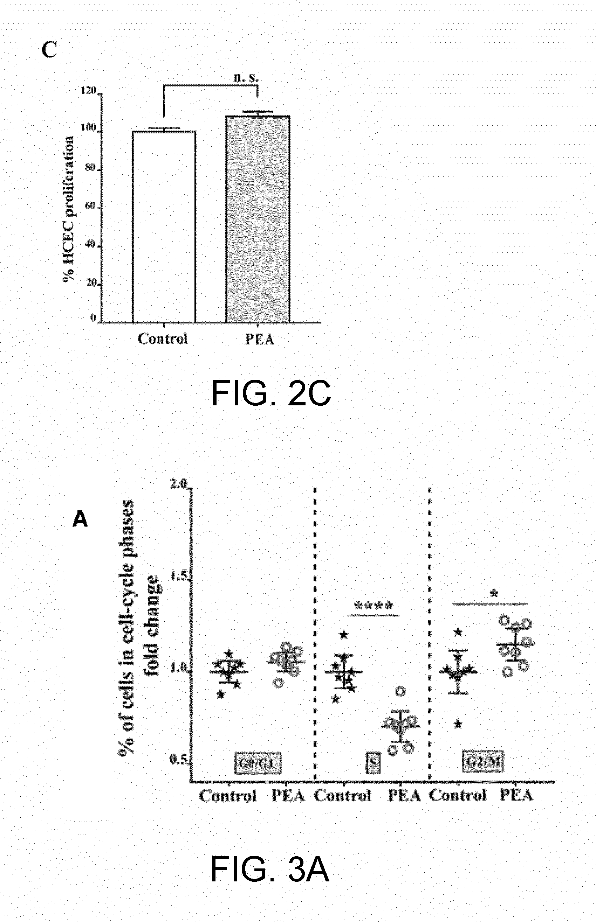

Figures 2A, 2B and 2C depict graphs of the cell proliferation rate of HCT116 (A), Caco-2 (B) cells and healthy human colon epithelial cells HCEC (C) in the presence or in the absence of ultra-micronized PEA for 24h, in which the results are expressed as a cell proliferation percentage as averages ±SEM; *P<0.05 and ***P0<.001 with respect to the control group and evaluated with Student's t test (ns = not significant);

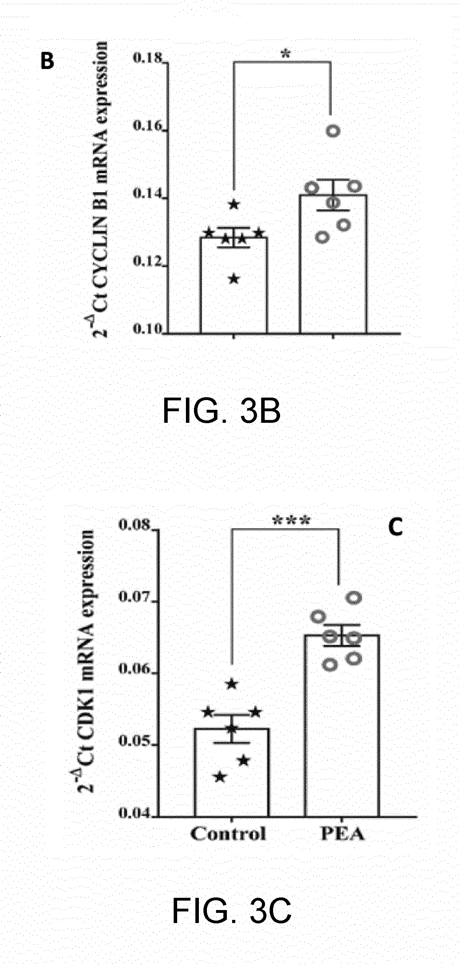

Figures 3A, 3B and 3C depict graphs of the induction of cell cycle arrest by ultra-micronized PEA: (A) Cell cycle analysis by flow cytometry of HCT116 cells in the presence or absence of ultra-micronized PEA (30 µM for 24h), *P<0.05 and ****P<0.0001 vs. control evaluated with Student's t-test; Gene expression of cyclin B1 (B) and cyclic-dependent kinase 1 (CDK1) (C) in HCT116 cells, in the presence or absence of ultra-micronized PEA (30 µM for 24h); the expression of the genes was measured with Real-Time PCR and the results are expressed as averages ± SEM; *P<0.05 and ***P0<.001 with respect to the control group and evaluated with Student's t test;

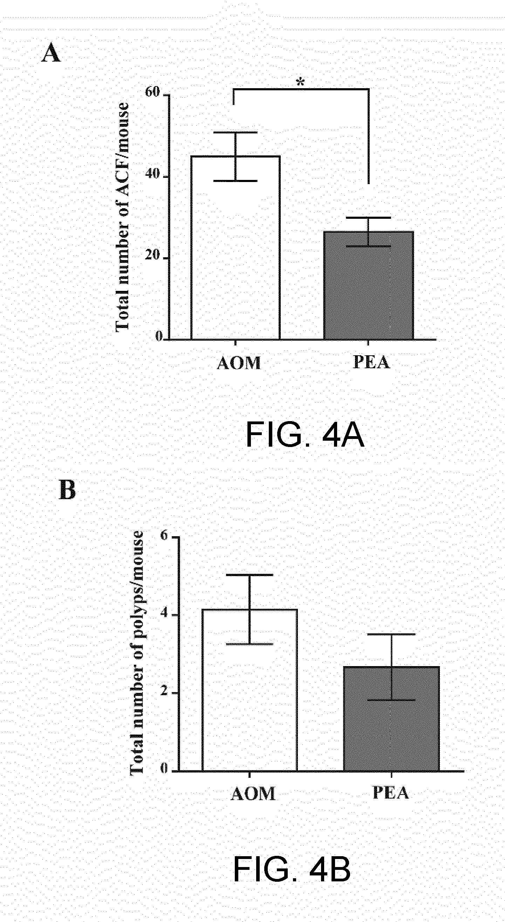

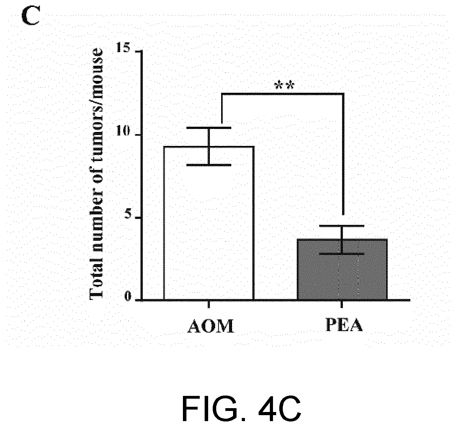

Figures 4A, 4B and 4C depict graphs of the effect of ultra-micronized PEA treatment in the AOM mouse model of colon cancer: (A) total number of ACFs, (B) polyps and (C) tumors derived from the administration of AOM (10 mg/kg at the beginning of the first, second, third and fourth week of experimentation corresponding to 40 mg/kg total intraperitoneally) in mice treated or not treated with ultra-micronized PEA 10 mg/kg *per os* three times a week for the duration of the trial (13 weeks) starting the week before the first administration of AOM to appreciate the chemo-preventive effect thereof; the results are expressed as averages ±SEM; *p<0.05 and **p<0.01 with respect to the group treated with AOM alone using Student's t-test.

### Detailed description of the invention

The present invention relates in a first aspect to palmitoylethanolamide (PEA) for use in the prevention of colorectal carcinoma (CRC), as defined in the claims.

The term "prevention" means the pharmacological treatment of a healthy patient, preferably a patient at risk of colorectal cancer, in order to hinder the onset of a neoplastic alteration or the neoplastic transformation of a benign formation by acting on both the activation phase and on proliferation.

In particular, by virtue of the present invention, patients at risk of CRC subjected to the search for tumor and inflammatory biomarkers and which test positive for such biomarkers, can be put under chronic PEA therapy, in particular ultra-micronized PEA, to restore the correct intestinal environment in order to counteract and/or slow down the onset of CRC.

In a non-claimed aspect, the present disclosure relates to palmitoylethanolamide (PEA) for use in the antineoplastic therapy of CRC, in which said PEA exerts a selective antiproliferative action on tumor cells and not on healthy cells.

In particular, in a non-claimed aspect, the disclosure relates to palmitoylethanolamide (PEA) for use in the antineoplastic therapy of CRC, in which said PEA exerts a selective antiproliferative action on tumor cells by stopping the cell cycle of neoplastic cells in the G2/M phase.

Preferably, the PEA is in ultra-micronized form.

The term "palmitoylethanolamide (or PEA) in ultra-micronized form" means PEA having a particle size distribution, defined as a percentage by volume and measured with the laser light scattering method, represented by a distribution curve having the mode below 6 microns and above 0.5 microns, measured with a Malvern Mastersizer 3000 instrument with Fraunhofer calculation algorithm.

In an embodiment, the PEA in ultra-micronized form has a particle size distribution as defined above, measured with a Malvern Mastersizer 3000 instrument with Fraunhofer calculation algorithm, where at least 95% by volume, more preferably at least 99% by volume, of particles has a particle size of less than 6 microns.

In a particularly preferred embodiment, the PEA in ultra-micronized form has a particle size distribution as defined above, measured with a Malvern Mastersizer 3000 instrument with Fraunhofer calculation algorithm, having a mode between 2 and 4 microns and having 100% by volume of particles smaller than 10 microns and at least 60% by volume of particles smaller than 3 microns.

The micronization can be carried out in a fluid jet system (for example, Jetmill^{®} model system) which operates with spiral technology with a compressed air or nitrogen jet capable of exploiting kinetic energy - instead of mechanical energy - to crush the particles. Such apparatuses are conventional and will therefore not be further described, except in relation to the following features:
- Internal diameter of the micronization chamber about 300 mm;
- Fluid jet pressure 10-12 bar;
- Product supply 9-12 kg/h.

For the purposes of the invention, the PEA is included in pharmaceutical or veterinary formulations and is formulated in dosage forms for oral administration.

For oral administration, the pharmaceutical compositions can be, for example, in the form of tablets or capsules, hard or soft, prepared in the conventional fashion with pharmaceutically acceptable excipients such as binding agents (e.g., pregelatinized cornstarch, polyvinylpyrrolidone or methylcellulose hydroxypropyl); filling agents (e.g., lactose, microcrystalline cellulose or calcium hydrogen phosphate); lubricants (e.g., magnesium stearate, talc or silica); disintegrants (e.g., potato starch or sodium starch glycolate); or inhibiting agents (e.g. sodium lauryl sulfate). The tablets can be coated with the methods well known in the art. The liquid preparations for oral administration can be, for example, in the form of solutions, syrups or suspensions or they can be freeze-dried or granulated products to be reconstituted, before use, with water or other suitable vehicles. Such liquid preparations can be prepared through conventional methods with pharmaceutically acceptable additives such as suspending agents (e.g., sorbitol syrup, cellulose derivatives, or edible hydrogenated fats); emulsifying agents (e.g., lecithin or acacia); non-aqueous vehicles (e.g., almond oil, oily esters, ethyl alcohol or fractionated vegetable oils); and preservatives (e.g., methyl- or propyl-p-hydroxybenzoates or sorbic acid). The preparation can also conveniently contain flavorings, dyes, and sweetening agents.

The preparations for oral administration can be formulated appropriately to allow the controlled release of the active constituent.

For buccal administration (not part of the claimed invention), the compositions can be in the form of tablets or pills formulated in the conventional fashion, adapted to an absorption at the level of the buccal mucosa. Typical buccal formulations are tablets for sub-lingual administration.

In a non-claimed aspect, the compositions can be formulated for parenteral administration by injection. The injection formulations can be presented as a single dose, for example in vials, with an added preservative. The compositions can appear in this form as suspensions, solutions, or emulsions in oily or aqueous vehicles and can contain agents of the formulation such as suspension, stabilizing and/or dispersing agents. Alternatively, the active constituent can be in the form of a powder to be reconstituted, before use, with a suitable vehicle, for example with sterile water.

In another non-claimed aspect, the compositions can also be formulated according to rectal formulations such as suppositories or retention enemas, for example containing the basic components of the common suppositories such as cocoa butter or other glycerides.

In addition to the compositions described above, in a non-claimed aspect, the compositions can also be formulated as a deposit preparation. Such long-acting formulations can be administered by implantation (e.g., subcutaneously, transcutaneously or intramuscularly) or by intramuscular injection. Thus, for example, the composition can be formulated with appropriate polymeric or hydrophobic materials (for example in the form of an emulsion in a suitable oil) or ion exchange resins or as minimally soluble derivatives.

According to the present invention, the dose of PEA proposed for administration to a human (with a body weight of about 70 kg) ranges from 10 mg to 1500 mg or from 100 mg to 900 mg of PEA per dose unit. The dose unit can be administered, for example, 1 to 4 times a day. The dose will depend on the form in which the PEA is administered, i.e., whether non-micronized PEA, micronized PEA or ultra-micronized PEA is administered. The dose will also depend on the route chosen for administration. It should be considered that it may be necessary to continuously vary the dosage depending on the age and weight of the patient and also on the severity of the clinical condition to be treated. The exact dose and route of administration will ultimately be at the discretion of the attending physician or veterinarian.

For a preventive treatment, the PEA can be administered for prolonged periods or for a chronic preventive treatment, by virtue of the very low toxicity thereof.

The invention further relates to dietary compositions, food supplements and foods for special medical purposes (FSMPs) comprising PEA, preferably ultra-micronized PEA for use in the prevention of the onset of CRC as defined in the claims in a healthy subject.

The term "food for special medical purposes" means products authorized according to regulation (EU) 2016/128. Such a term refers to a product to be administered under medical supervision, thus assimilating such an FSMP to a drug.

The formulations according to the invention can be prepared according to conventional methods, such as those described in Remington's Pharmaceutical Sciences Handbook, Mack Pub. Co., N.Y., USA, 17th edition, 1985.

### EXPERIMENTAL PART

### Micronization procedure

The PEA was micronized as previously described.

The ultra-micronization was carried out in a fluid jet system (in particular, the Jetmill^{®} model system) which operates with compressed air jet "spiral technology".

Optimal micronization conditions:
- internal diameter of the micronization chamber 300 mm;
- fluid jet pressure 8 bar;
- product supply 9-12 kg/h.

### Determination of the particle size distribution

The determination of the particle size distribution was carried out on a wet sample, after 1-minute sonication.

A Malvern Mastersizer 3000 instrument operating with the LALLS (Low Angle Laser Light Scattering) technique and a Fraunhofer calculation algorithm was used.

The particle size distribution graph is shown in figure 1.

### Biological experimentation

For the *in vitro* experiments, immortalized human colon cancer (HCT116) and human colorectal adenocarcinoma cell lines (Caco-2) and immortalized healthy human colon epithelial cells (HCEC) were cultured in DMEM growth medium (Sigma Aldrich) supplemented with 10% serum bovine fetal (FBS, Sigma Aldrich) and kept at 37°C in an incubator with a 5% CO₂ atmosphere.

To assess viability, the cells are stained with Trypan blue while cell proliferation, in the presence or absence of ultra-micronized PEA (1-30 µM), was determined using the BrdU proliferation ELISA kit (Roche, Milan, Italy).

The cell cycle analysis of HCT116 cells was performed following the protocol reported in the BD Pharmingen^{™} BrdU Flow Kit (BD Biosciences, USA). For the study, 1.5×10⁵ cells was sown on 6-well plates and kept overnight in serum-free medium in the presence or absence of ultra-micronized PEA (30µM) for 24h. The cells were detected with the BriCyte flow cytometer (Mindray, Italy) according to the BrdU (bromodeoxyuridine) and 7-AAD (7-aminoactinomycin D) content thereof. The data obtained were analyzed with FlowJo v10 software (tree Star, USA) to understand the distribution of HCT116 in the different cell cycle phases.

The expression of the CDK1 and CYCLIN B1 genes was evaluated by virtue of the quantitative Real-Time PCR method using specific primers.

The *in vivo* study was conducted on six-week-old (25-30g) male CD1 mice fed *ad libitum* and housed in cages with a controlled sleep/wake cycle. Before the start of the experimentation, the animals were subjected to an acclimatization period of 1 week considering all the experimental procedures and protocols, compliant with the principles of the care of laboratory animals approved by the Italian Ministry of Health and respecting the ARRIVE guidelines [Curtis MJ et al. Experimental design and analysis and their reporting II: updated and simplified guidance for authors and peer reviewers. Br J Pharmacol, 2018; 175: 987-993].

The animals were chemically treated with AOM (azoxymethane, 40 mg/kg in total) [Neufert C et al. An inducible mouse model of colon carcinogenesis for the analysis of sporadic and inflammation-driven tumor progression. Nat Protoc 2007; 2: 1998-2004] to induce the formation of the CRC. A single dose of AOM was injected intraperitoneally at a concentration of 10 mg/kg at the beginning of the first, second, third and fourth weeks of the experiment [Pagano E et al. Pharmacological inhibition of MAGL attenuates experimental colon carcinogenesis, Pharmacol Res 2017; 119: 227-236].

The animals were randomized into 2 groups of 7 animals each:
Group 1 was treated *per* os with 2% carboxymethylcellulose (CMC), vehicle used to suspend the ultra-micronized PEA.
Group 2 was treated *per os* with ultra-micronized PEA in 2% CMC at a concentration of 10 mg/kg three times a week for the duration of the experiment, starting the week before the first administration of AOM to appreciate the chemo-preventive effect thereof [Pagano E et al. Pharmacological inhibition of MAGL attenuates experimental colon carcinogenesis, Pharmacol Res 2017; 119: 227-236].

The animals were euthanized 12 weeks after the first AOM injection and the colorectal area was collected.

Based on laboratory experience, the timing and dose of AOM used ensure the appearance of a significant number of ACF, polyps and tumors [Izzo AA et al. Increased endocannabinoid levels reduce the development of precancerous lesions in the mouse colon. J Mol Med (Berl) 2008; 86: 89-98].

The detection and quantification of ACF, polyps and colorectal tumors was performed following the protocol of Pagano E et al. Pharmacological inhibition of MAGL attenuates experimental colon carcinogenesis, Pharmacol Res 2017; 119: 227-236.

### Results

### Proliferation of HCT116 and Caco-2 cells in vitro (for reference)

Ultra-micronized PEA significantly reduces the proliferation of HCT116 and Caco-2 cells *in vitro;* specifically, ultra-micronized PEA, used at increasing doses (1-30 µM), reduces the proliferation rate of HCT116 cells during the 24 hours of exposure (Figure 2A). Furthermore, 30 µM ultra-micronized PEA is capable of significantly reducing the proliferation of non-metastatic Caco-2 cells (Figure 2B). Surprisingly, the anti-proliferative effects of the ultra-micronized PEA did not affect the proliferation rate of the healthy epithelial cell line HCEC (Figure 2C), effectively showing a selective effect on tumor cells. This datum is very important because it allows a considerable reduction of side effects.

### Cell cycle arrest in the G2/M phase of HCT116 cells in vitro (for reference)

Flow cytometry revealed that the treatment of the HCT116 cells with ultra-micronized PEA (30 µM for 24h) is capable of blocking neoplastic cells in the G2/M phase with a concomitant significant reduction in the percentage of cells in the S phase (Figure 3A). Since it is known that the G2/M transition is regulated by the cyclin B1/CDK1 complex [Malumbres M and Barbacid M, 2005], we explored the behavior of such a complex in the presence of ultra-micronized PEA: we found that the treatment of cells with ultra-micronized PEA induces increased expression of the cyclin B1/CDK1 complex (Fig. 3B-C). These data demonstrate the ability of ultra-micronized PEA to actively participate not only in the arrest of neoplastic cells in the G2/M phase but also in the activation of the cyclin B1/CDK1 complex necessary for the arrest of the cell cycle in the aforesaid phase.

### Prevention of tumor development in the animal model of CRC (according to the invention)

Administration of the carcinogen AOM (azoxymethane) induces the formation of several foci of aberrant crypts, known as ACF (Figure 4A), polyps (Figure 4B) and tumors (Figure 4C). The treatment of animals with ultra-micronized PEA 10 mg/kg, administered *per os* three times a week for the duration of the experiment (13 weeks), starting the week before the first AOM administration, significantly reduces the number of pre-neoplastic ACF lesions (Figure 4A) and the total number of tumors (Figure 4C) induced by the carcinogen AOM. The ultra-micronized PEA showed a strong tendency to also reduce the number of polyps (Figure 4B).

In light of these results, the preventive administration of ultra-micronized PEA appears to be effective in reducing the development of CRC by virtue of the slowing of the transformation of intestinal mucosal cells into tumor cells (chemo-preventive effect). This datum demonstrates how the preventive intake of ultra-micronized PEA, in addition to being completely safe, significantly attenuates the aggressiveness of CRC, effectively improving life expectancy.

Furthermore, the slow transformation of mucosal cells allows, at the time of diagnosis, to treat a small CRC and, most likely, in the early phases of not yet aggressive staging.

The data obtained show, for the first time, how chemo-preventive treatment with PEA and in particular with ultra-micronized PEA, is capable of reducing the development of CRC.

The invention will now be further described by means of the following formulation examples.

### Formulation examples

### PEA-UM = Ultra-micronized palmitoylethanolamide

### Example 1

Each tablet contains:

| | |
|---|---|
| - PEA-UM | 300.00 mg |
| - Macrocrystalline cellulose | 78.47 mg |
| - Sodium croscarmellose | 45.00 mg |
| - Polyvinylpyrrolidone | 10.00 mg |
| - Magnesium stearate | 4.0 mg |
| - Polysorbate 80 | 2.00 mg |

### Example 2

Each tablet contains:

| | |
|---|---|
| - PEA-UM | 600.00 mg |
| - Macrocrystalline cellulose | 156.94 mg |
| - Sodium croscarmellose | 90.00 mg |
| - Polyvinylpyrrolidone | 20.00 mg |
| - Magnesium stearate | 8.00 mg |
| - Polysorbate 80 | 4.00 mg |

### Example 3

A 5 g dose of orodisintegrating microgranules contains:

| | |
|---|---|
| - PEA-UM | 500.00 mg |
| - Non-cariogenic sugar | 200.00 mg |
| - acceptable excipients as needed to | 5.00 g |

### Example 4

A hard gelatin capsule made gastro-resistant with an adequate coating contains:

| | |
|---|---|
| - PEA-UM | 400 mg |
| - Soy lecithin | 100 mg |
| - Lactose | 80 mg |

### Example 5

A tablet coated with a gastro-resistant layer contains:

| | |
|---|---|
| -PEA-UM | 500 mg |
| -Micronized PEA | 250 mg |
| -Polyvinylpyrrolidone | 30 mg |
| -Sodium croscarmellose | 80 mg |
| -Magnesium stearate | 7 mg |

### Example 6 (reference)

A suppository contains:

| | |
|---|---|
| -PEA-UM | 300 mg |
| -Non-micronized PEA | 500 mg |
| -Lipophilic mass for suppositories as needed to | 3 g |

### Example 7 (reference)

A 2.5 ml dose of oily gel contains

| | |
|---|---|
| - PEA-UM | 600.00 mg |
| - Glyceryl monostearate | 40.00 mg |
| - Vegetable oil as needed to | 2.50 ml. |

## Claims

1. Palmitoylethanolamide for use in the prevention of colorectal carcinoma, adenomatous polyps and/or pre-neoplastic ACF (Aberrant Crypts Foci) lesions, wherein said palmitoylethanolamide is acting on both the activation and proliferation phase and wherein palmitoylethanolamide is contained in a pharmaceutical or veterinary formulation or it is contained in dietary compositions, food supplements, or foods for special medical purposes (FSMPs), and is formulated in dosage forms for oral administration.

2. Palmitoylethanolamide for use according to claim 1, wherein palmitoylethanolamide is in ultra-micronized form having a particle size distribution, defined as percentage by volume and measured by the laser light scattering method, represented by a distribution curve having the mode below 6 microns and above 0.5 microns, measured with a Malvern Mastersizer 3000 instrument with Fraunhofer calculation algorithm.

3. Palmitoylethanolamide for use according to claim 2, having a particle size distribution, defined as percentage by volume and measured by the laser light scattering method, measured with a Malvern Mastersizer 3000 instrument with Fraunhofer calculation algorithm, wherein at least 95% by volume, preferably at least 99% by volume, of particles has a particle size less than 6 microns.

4. Palmitoylethanolamide for use according to claim 2, wherein the palmitoylethanolamide has a particle size distribution, defined as percentage by volume and measured by the laser light scattering method, measured with a Malvern Mastersizer 3000 instrument with Fraunhofer calculation algorithm, having a mode between 2 and 4 microns and having 100% by volume of particles smaller than 10 microns and at least 60% by volume of particles smaller than 3 microns.

5. Palmitoylethanolamide for use according to any one of claims 1 to 4, wherein palmitoylethanolamide is administered in doses of 10 mg to 1500 mg or 100 mg to 900 mg of PEA per dose unit, 1 to 4 times per day.

## Patentansprüche

1. Palmitoylethanolamid zur Verwendung in der Prävention von kolorektalem Karzinom, adenomatösen Polypen und/oder präneoplastischen ACF (aberranten Kryptenherden)-Läsionen, wobei das Palmitoylethanolamid sowohl auf die Aktivierungs- als auch auf die Proliferationsphase wirkt und wobei Palmitoylethanolamid in einer pharmazeutischen oder veterinärmedizinischen Formulierung enthalten ist oder es in diätetischen Zusammensetzungen, Nahrungsergänzungsmitteln oder Lebensmitteln für besondere medizinische Zwecke (FSMPs) enthalten ist und in Dosierungsformen für orale Verabreichung formuliert ist.

2. Palmitoylethanolamid zur Verwendung nach Anspruch 1, wobei Palmitoylethanolamid in ultramikronisierter Form vorliegt, mit einer Partikelgrößenverteilung, definiert als Volumenprozent und gemessen durch das Laserlichtstreuungsverfahren, dargestellt durch eine Verteilungskurve mit dem Modus unter 6 Mikrometer und über 0,5 Mikrometer, gemessen mit einem Malvern Mastersizer 3000 Instrument mit Fraunhofer-Berechnungsalgorithmus.

3. Palmitoylethanolamid zur Verwendung nach Anspruch 2, mit einer Partikelgrößenverteilung, definiert als Volumenprozent und gemessen durch das Laserlichtstreuungsverfahren, gemessen mit einem Malvern Mastersizer 3000 Instrument mit Fraunhofer-Berechnungsalgorithmus, wobei mindestens 95 Volumen-%, bevorzugt mindestens 99 Volumen-%, der Partikel eine Partikelgröße von weniger als 6 Mikrometer aufweisen.

4. Palmitoylethanolamid zur Verwendung nach Anspruch 2, wobei das Palmitoylethanolamid eine Partikelgrößenverteilung, definiert als Volumenprozent und gemessen durch das Laserlichtstreuungsverfahren, gemessen mit einem Malvern Mastersizer 3000 Instrument mit Fraunhofer-Berechnungsalgorithmus, mit einem Modus zwischen 2 und 4 Mikrometern und mit 100 Volumen-% der Partikel kleiner als 10 Mikrometer und mindestens 60 Volumen-% der Partikel kleiner als 3 Mikrometer.

5. Palmitoylethanolamid zur Verwendung nach einem der Ansprüche 1 bis 4, wobei Palmitoylethanolamid in Dosen von 10 mg bis 1500 mg oder 100 mg bis 900 mg von PEA pro Dosiseinheit 1 bis 4 Mal pro Tag verabreicht wird.

## Revendications

1. Palmitoyléthanolamide pour son utilisation dans la prévention du carcinome colorectal, de polypes adénomateux et/ou de lésions prénéoplasiques ACF (foyers de cryptes aberrantes ou « Aberrant Crypts Foci »), dans lequel ledit palmitoyléthanolamide agit à la fois sur la phase d'activation et la phase de prolifération et dans lequel le palmitoyléthanolamide est contenu dans une formulation pharmaceutique ou vétérinaire ou il est contenu dans des compositions diététiques, des compléments alimentaires, ou des denrées alimentaires destinées à des fins médicales spéciales (DADFMS), et est formulé sous forme posologique pour une administration orale.

2. Palmitoyléthanolamide pour utilisation selon la revendication 1, dans lequel le palmitoyléthanolamide est sous forme ultra micronisée présentant une distribution granulométrique, définie en pourcentage en volume et mesurée par le procédé de diffusion de lumière laser, représentée par une courbe de distribution présentant le mode inférieur à 6 micromètres et supérieur à 0,5 micromètre, mesurée avec un instrument Mastersizer 3000 de Malvern avec un algorithme de calcul de Fraunhofer.

3. Palmitoyléthanolamide pour utilisation selon la revendication 2, présentant une distribution granulométrique, définie en pourcentage en volume et mesurée par le procédé de diffusion de lumière laser, mesurée avec un instrument Mastersizer 3000 de Malvern avec un algorithme de calcul de Fraunhofer, dans lequel au moins 95 % en volume, de préférence au moins 99 % en volume, de particules présentent une taille de particule inférieure à 6 micromètres.

4. Palmitoyléthanolamide pour utilisation selon la revendication 2, dans lequel le palmitoyléthanolamide présente une distribution granulométrique, définie en pourcentage en volume et mesurée par le procédé de diffusion de lumière laser, mesurée avec un instrument Mastersizer 3000 de Malvern avec un algorithme de calcul de Fraunhofer, présentant un mode entre 2 et 4 micromètres et présentant 100 % en volume de particules inférieures à 10 micromètres et au moins 60 % en volume de particules inférieures à 3 micromètres.

5. Palmitoyléthanolamide pour utilisation selon l'une quelconque des revendications 1 à 4, dans lequel le palmitoyléthanolamide est administré à des doses de 10 mg à 1 500 mg ou 100 mg à 900 mg de PEA par dose unitaire, 1 à 4 fois par jour.
